**Europäisches Patentamt**

**European Patent Office**

**Office européen des brevets**

(19)

(11) Veröffentlichungsnummer : **0 100 047**
**B1**

(12) **EUROPÄISCHE PATENTSCHRIFT**

(45) Veröffentlichungstag der Patentschrift :
12.02.86

(51) Int. Cl.⁴ : **C 07 C119/042, C 07 C118/00**

(21) Anmeldenummer : **83107019.8**

(22) Anmeldetag : **18.07.83**

(54) **Verfahren zur Herstellung von Hexamethylen-diisocyanat-1,6 und/oder isomeren Diisocyanaten mit 6 Kohlenstoffatomen im Alkylenrest.**

(30) Priorität : **24.07.82 DE 3227748**
**24.12.82 DE 3248018**

(43) Veröffentlichungstag der Anmeldung :
**08.02.84 Patentblatt 84/06**

(45) Bekanntmachung des Hinweises auf die Patenterteilung : **12.02.86 Patentblatt 86/07**

(84) Benannte Vertragsstaaten :
**BE DE FR GB IT NL**

(56) Entgegenhaltungen :
**DE-A- 2 512 514**
**DE-A- 3 108 990**
**FR-A- 1 160 218**
**GB-A-   574 222**
**GB-A- 1 247 451**
**US-A- 2 409 712**
**US-A- 3 631 198**

(73) Patentinhaber : **BASF Aktiengesellschaft**
**Carl-Bosch-Strasse 38**
**D-6700 Ludwigshafen (DE)**

(72) Erfinder : **Merger, Franz, Dr.**
**Max-Slevogt-Strasse 25**
**D-6710 Frankenthal (DE)**
Erfinder : **Nestler, Gerhard, Dr.**
**Van-Leyden-Strasse 17**
**D-6700 Ludwigshafen (DE)**
Erfinder : **Towae, Friedrich, Dr.**
**Parkstrasse 22**
**D-6700 Ludwigshafen (DE)**
Erfinder : **Hellbach, Hans**
**Stettiner Strasse 14**
**D-6840 Lampertheim (DE)**

## Beschreibung

Die technische Herstellung von Hexamethylen-diisocyanat-1,6 beruht auf der Phosgenierung von Hexamethylendiamin zu Hexamethylen-dicarbamidsäurechlorid und dessen thermische Spaltung in Hexamethylen-diisocyanat-1,6 und Chlorwasserstoff. Abgesehen von den schwerwiegenden Umweltschutz- und Sicherheitsproblemen, die der Einsatz von Phosgen mit sich bringt, ist dieses Verfahren noch mit weiteren entscheidenden Nachteilen behaftet. So gelingt die Hexamethylen-diisocyanat-1,6-Produktion nur mit recht mäßigem Raum-Zeit-Ausbeuten. Neben Hexamethylen-diisocyanat-1,6 erhält man außerdem mehrere Nebenprodukte, von denen das wichtigste — 6-Chlorhexylisocyanat — zudem den Nachteil besitzt, daß es nur mit erheblichem destillativen Aufwand vom Hexamethylen-diisocyanat-1,6 abgetrennt werden kann.

Es ist bekannt, daß N-substituierte aromatische Urethane thermisch in der Gasphase oder in flüssiger Phase in Isocyanate spaltbar sind. Bei der thermischen Spaltung treten jedoch vielfach zusätzlich verschiedene unerwünschte Nebenreaktionen auf. Genannt seien beispielsweise die Decarboxylierungsreaktion der Urethane, die von der Bildung primärer und sekundärer Amine sowie von Olefinen begleitet sein kann ; die Umsetzungen zwischen dem gebildeten Isocyanat und Urethan zu Allophanaten bzw. Amin zu Harnstoffen und die Polymerisation der Isocyanate zu Isocyanuraten.

Die Pyrolyse der Urethane in der Dampfphase wird nach Angaben der DE-AS 19 44 719 (GB-PS 1 247 451) bei Temperaturen von 400 bis 600 °C in Gegenwart von Lewissäure als Katalysator durchgeführt, wobei das Isocyanat und der Alkohol durch fraktionierte Kondensation getrennt werden. 2,4-Toluylendiisocyanat wird z. B. durch Pyrolyse von Toluylen-2,4-diethylurethan in Gegenwart von Eisen-(III)-chlorid erhalten. Nachteile der Reaktion sind u. a. niedrige Ausbeuten, verbunden mit beträchtlichen Mengen eines polymeren Nebenproduktes, Zersetzung des Katalysators und Korrosion der Reaktionsapparatur. In der DE-OS 24 10 505 (US 3 870 739) wird ein Verfahren beschrieben, bei dem ein aromatisches Urethan bei einer Temperatur von 350 bis 550 °C und einem Druck von weniger als dem (m + 1) fachen des Isocyanatdampfdruckes in einer katalysatorfreien Pyrolysezone innerhalb von 15 Sekunden gespalten wird. Nachteilig an diesem Verfahren ist u. a., daß ein als Nebenprodukt anfallendes festes Polymer und dessen Abtrennung die Durchführung eines kontinuierlichen Verfahrens erschwert.

Zur Herstellung von aromatischen Isocyanaten werden Urethane nach DE-OS 26 35 490 (US 4 081 472) bei Temperaturen von 150 bis 350 °C unter vermindertem Druck mit einer Lösung aus wenigstens einem Metallion, wie Ionen von Kupfer, Zink, Aluminium, Zinn, Titan, Vanadium, Eisen, Cobalt und Nickel als Katalysator, der in einem Lösungsmittel mit einem Siedepunkt von 200 °C in einer Metallkonzentration von wenigstens 0,001 Gew.-%, bezogen auf das Lösungsmittel, gelöst ist, in Kontakt gebracht. Die Trennung der erhaltenen Spaltprodukte erfolgt durch fraktionierte Kondensation. Hierbei bleiben jedoch in geringen Mengen gebildete, nicht destillierbare Polymerisationsprodukte im Katalysator enthaltenden Lösungsmittelrückstand, wodurch nach einiger Zeit zusätzlich Reinigungsmittel notwendig werden.

Nach DE-OS 29 42 543 (US 4 330 479) erzielt man sehr gute Spaltergebnisse, wenn man aromatische Urethane an katalytisch wirksamen, oberflächenreichen Metallen, die in heterogener Phase vorliegen, spaltet. Nachteilig an diesem Verfahren ist, daß die als Katalysatoren eingesetzten Metalle mit der Zeit durch Belegung ihre katalytische Aktivität verlieren, wodurch ebenfalls zusätzlich Reinigungsoperationen notwendig werden.

Zur Beseitigung dieses Mangels wird nach Angaben der deutschen Offenlegungsschrift 31 42 627 die thermische Spaltung in Gegenwart von Kohlenstoff, der vorzugsweise als Fettstoffschüttung in einem Wirbelbett vorliegt, durchgeführt. Vorteilhaft ist hierbei, daß der durch polymere Neben- oder Zersetzungsprodukte desaktivierte Katalysator nicht regeneriert zu werden braucht, sondern durch Verbrennen rückstandsfrei und umweltfreundlich vernichtet werden kann. Die Ausbeuten an Hexamethylendiisocyanat liegen jedoch durchweg unter 90 %.

Die Ausführungen zeigen, daß die bekannten Herstellungsverfahren für Isocyanate teilweise noch erhebliche Mängel aufweisen. Da in den Beispielen der genannten Publikationen nahezu ausschließlich die Spaltung von aromatischen Urethanen beschrieben wird, legt dies den Schluß nahe, daß auf diese Weise aliphatische Diisocyanate technisch nicht befriedigend produziert werden können.

Aufgabe der vorliegenden Erfindung war es, ein verbessertes, kostengünstiges und umweltfreundliches Verfahren zur Herstellung von Hexamethylen-diisocyanat-1,6 und/oder isomeren aliphatischen Diisocyanaten mit 6 Kohlenstoffatomen im Alkylenrest — im folgenden zusammenfassend kurz Diisocyanat genannt — zu entwickeln.

Überraschenderweise wurde gefunden, daß man Hexamethylendialkylurethane und/oder die entsprechenden aliphatischen Isomere unter bestimmten Reaktionsbedingungen in technisch einfacher Weise und in sehr guten Ausbeuten thermisch in Diisocyanat spalten kann.

Gegenstand der Erfindung ist somit ein Verfahren zur Herstellung von Hexamethylen-diisocyanat-1,6 und/oder isomeren aliphatischen Diisocyanaten mit 6 Kohlenstoffatomen im Alkylenrest durch thermische Spaltung von Hexamethylen-dialkylurethanen-1,6 und/oder isomeren aliphatischen Dialkylurethanen mit 6 Kohlenstoffatomen im Alkylenrest, das dadurch gekennzeichnet ist, daß man die Dialkylurethane mit 1 bis 8 Kohlenstoffatomen im Alkylrest bei Temperaturen von 220 bis 300 °C und unter einem Druck von 0,1 bis 200 mbar in einem Verdampfer unzersetzt verdampft, bei Temperaturen über 300 °C in der Gasphase unter vermindertem Durck thermisch spaltet und die hierbei gebildeten Spaltgase fraktionierend kondensiert.

Nach einer bevorzugten Ausführungsform wird die thermische Spaltung in Gegenwart von Halogenwasserstoffen und/oder Halogenwasserstoff-Donatoren, die unter den Reaktionsbedingungen Halogenwasserstoff bilden, durchgeführt.

Als isomere aliphatische Diisocyanate mit 6 Kohlenstoffatomen im Alkylenrest seien insbesondere 2-Methyl-pentamethylen-1,5-diisocyanat und 2-Ethyl-tetramethylen-1,4-diisocyanat genannt. Das erfindungsgemäße Verfahren findet somit vorzugsweise Anwendung zur Herstellung der zwei obengenannten Isomeren und insbesondere von Hexamethylen-diisocyanat-1,6 sowie Gemischen aus diesen Diisocyanaten.

Als Ausgangsprodukte für das erfindungsgemäße Verfahren eignen sich prinzipiell alle Hexamethylen-dialkylurethane und/oder isomeren aliphatischen Dialkylurethane mit 6 Kohlenstoffatomen im Alkylenrest — im folgenden zusammenfassend kurz Diurethan genannt — mit 1 bis 8 Kohlenstoffatomen im Alkylrest oder deren Gemische. Bevorzugt verwendet werden jedoch Ausgangsprodukte, bei denen die folgenden Forderungen erfüllt sind :

a) das Diurethan muß unzersetzt verdampfbar sein,

b) die Siedepunkte der Spaltprodukte Diisocyanat und Alkohol müssen so weit auseinanderliegen, daß durch fraktionierende Kondensation eine möglichst quantitative Trennung der beiden Endprodukte erreicht wird :

c) um Materialverluste zu vermeiden, muß der abgespaltene Alkohol in der 2. Kondensationsstufe möglichst quantitativ mit technisch realistischen Methoden kondensierbar sein.

Die genannten Forderungen werden beispielsweise erfüllt durch Diurethane mit 1 bis 5 C-Atomen im Alkylrest, wie z. B. Hexamethylendimethylurethan, Hexamethylendiethylurethan, Hexamethylendipropylurethan, Hexamethylen-di-n-butylurethan, Hexamethylendi-iso-butylurethan, Hexamethylendi-n-pentylurethan, Hexamethylendi-isopentylurethan sowie den entsprechenden 2-Methyl-pentamethylen-1,5- und-/oder 2-Ethyl-tetramethylen-1,4-dialkylurethan-Isomeren, so daß diese Diurethane vorzugsweise Anwendung finden. Ganz besonders vorteilhafte Ergebnisse wurden bei der thermischen Spaltung von Hexamethylen-1,6-, 2-Methyl-pentamethylen-1,5- und 2-Ethyl-tetramethylen-1,4-di-n-butylurethan und-/oder -di-isobutylurethan erzielt, so daß insbesondere diese Dialkylurethane eingesetzt werden. Die Diurethane können nach bekannten Verfahren hergestellt werden. Phosgenfrei werden sie in sehr guten Ausbeuten beispielsweise durch Umsetzung von aliphatischen Diaminen mit 6 Kohlenstoffatomen im Alkylenrest mit Harnstoff oder Harnstoff-O-Butylcarbamat-Mischungen in Gegenwart von Butanol gemäß DE-OS 29 17 490 bzw. DE-OS 29 17 493 erhalten.

Wie bereits dargelegt wurde, wird die thermische Spaltung vorzugsweise in Gegenwart von Halogenwasserstoffen und/oder Halogenwasserstoff-Donatoren, die unter den Reaktions-bedingungen Halogenwasserstoff bilden, durchgeführt. Hierbei wird überraschenderweise kein 6-Chlor- oder 6-Bromhexylisocyanat gebildet. Außerdem hat sich gezeigt, daß die Standzeit der Reaktorfüllung erheblich verlängert und eine sehr gute Selektivität bezüglich Diisocyanat, insbesondere Hexamethylen-diisocyanat-1,6, über einen bedeuten längeren Zeitraum erreicht wird.

Als Halogenwasserstoffe werden vorzugsweise Bromwasserstoff und insbesondere Chlorwasserstoff verwendet. Geeignet sind jedoch auch Halogenwasserstoff-Donatoren, die unter den Reaktionsbedingungen der thermischen Spaltung die obengenannten Halogenwasserstoffe bilden. Als Halogenwasserstoff-Donatoren seien beispielhaft genannt : organische und/oder anorganische Säurehalogenide, wie z. B. Acetylchlorid, Propionylchlorid, Chlorkohlensäure-methylester, -ethylester, Chlorsulfonsäure, Bromcyan und Chlorcyan, Ammoniumhalogenide und/oder Aminhydrohalogenide von Aminen mit Siedepunkten kleiner als 150 °C, wie z. B. Ammonium-chlorid, -bromid, Trimethylaminhydrobromid, Diethylaminhydrochlorid, Propylaminhydrochlorid und Isoamylaminhydrobromid, und vorzugsweise Halogenalkane und/oder -alkene, wie z. B. Methylchlorid, Chlorethan, Bromethan, 1,2-Dichlorethan, 1,1,2-Trichlorethan, Vinylchlorid und Allylbromid.

Die Halogenwasserstoffe werden in Mengen von 0,001 bis 1,0 Mol-%, vurzugsweise von 0,002 bis 0,2 Mol-% pro Mol Diurethan eingesetzt. Verwendet man dagegen Halgoenwasserstoff-Donatoren, so kommen diese in solchen Mengen zur Anwendung, daß unter den Reaktionsbedingungen der thermischen Spaltung 0,001 bis 1,0 Mol-%, vorzugsweise 0,002 bis 0,2 Mol-% Halogenwasserstoff, beispielsweise Brom- oder insbesondere Chlorwasserstoff, gebildet werden.

Die Halogenwasserstoff-Donatoren oder vorzugsweise Halogenwasserstoffe können mit den Diurethanen gemischt und die Mischung in den Verdampfer eingebracht werden. Sie können jedoch auch den Diurethanen im Verdampfer einverleibt oder direkt in den Spaltreaktor während der thermischen Spaltung eindosiert werden. Nach der bevorzugten Ausführungsform werden Diurethan und Halogenwasserstoff oder -Donatoren vor der Spaltung gemischt und als Mischung dem Spaltreaktor zugeführt.

Die Diurethane können in flüssiger oder fester Form, z. B. als Schmelze oder als Pulver, oder aber auch als Suspension oder Lösung in einem unter den Reaktionsbedingungen inerten Lösungsmittel in den Verdampfer eingebracht werden.

Als Verdampfer, die bei Temperaturen von 220 bis 300 °C, vorzugsweise von 240 bis 280 °C und

# 0 100 047

unter einem Druck von 0,1 bis 200 mbar, vorzugsweise von 5 bis 100 mbar betrieben werden, haben sich insbesondere Dünnschichtverdampfer bewährt.

Durch eine ausreichende Wärmezufuhr ist es möglich, das gesamte eingebrachte Diurethan zu verdampfen. Es hat sich jedoch gezeigt, daß es von Vorteil ist, einen Teil des Diurethan unverdampft als Schmelze aus dem Verdampfer auszuschleusen, da man hierdurch einen Reinigungseffekt an der Verdampfverwand erzielt. Das Gewischtsverhältnis von verdampftem zu unverdampftem Diurethan ist hierbei in der Regel 20 : 80 bis 90 : 10, bevorzugt 40 : 60 bis 60 : 40.

Die Diurethan-Dämpfe werden anschließend in den Spaltreaktor eingeführt, indem die Spaltung bei Temperaturen über 300 °C, vorzugsweise 310 bis 480 °C und insbesondere 360 bis 440 °C diskontinuierlich oder kontinuierlich unter vermindertem Druck, vorzugsweise 0,1 bis 200 mbar, insbesondere 1 bis 100 mbar, in Gegenwart der Halogenwasserstoffe und gegebenenfalls von Inertgasen durchgeführt wird.

Der Spaltreaktor, der im allgemeinen säulenförmig ist, kann einen Querschnitt in beliebiger Form aufweisen. Vorzugsweise verwendet man langgestreckte, zylinderförmige Spaltreaktoren. Das Verhältnis von Innendurchmesser zu Länge des Spaltreaktors beträgt im allgemeinen 1 : 2 bis 1 : 1 000, vorzugsweise 1 : 10 bis 1 : 500. Die Spaltreaktoren können senkrecht oder waagrecht ausgerichtet sein und auch Zwischenlagen einnehmen. Vorzugsweise verwendet werden Röhrenofen als Spaltreaktoren, bei denen der Rohrinnendurchmesser etwa 10 bis 100 mm und die Rohrlänge ungefähr 0,5 bis 5 m beträgt.

Zweckmäßigerweise führt man die Spaltung in Gegenwart von thermisch stabilen Reaktorfüllkörpern durch. Als Füllkörper geeignet sind alle temperaturbeständigen und gasdurchlässigen Materialien, wie z. B. Perlen, Wolle, Ringe und/oder Späne aus Kohle, Stahl, Messing, Kupfer, Zink, Aluminium, Titan, Chrom, Kobalt, Nickel und/oder Quarz.

Einige dieser Materialien wie z. B. Stahl, Messing und insbesondere Aluminium, Zink und Kohlenstoff haben sich besonders bewährt und werden daher bevorzugt verwendet, da sie zu besseren Spaltergebnissen führen. Bewährt hat sich auch die Mitverwendung von Inertgasen, wie z. B. Stickstoff, als Verdünnungsmittel für die Halogenwasserstoffe und/oder als Strippmittel.

Aus dem Spaltreaktor führt man die in der Dampfphase befindlichen Dissoziationsprodukte, die nahezu ausschließlich aus Diisocyanat und Alkohol bestehen, in eine Zweistufen-Dampfkondensationsvorrichtung. In der ersten Kondensationsstufe, die abhängige vom Systemdruck von 0,1 bis 100 mbar bei Temperaturen von 60 bis 120 °C betrieben wird, kondensiert das Diisocyanat nahezu vollständig aus. Bei der bevorzugten Verwendung von 1,6-Hexamethylendibutylurethan wird beispielsweise bei einem Systemdruck von 20 bis 40 mbar zweckmäßigerweise eine Kondensationstemperatur von 70 bis 100 °C eingehalten. Die Temperatur der zweiten Kondensationsstufe richtet sich nach dem Siedepunkt des gebildeten Alkohols. Bei der Spaltung von 1,6-Hexamethylendibutylurethan beispielsweise wird zweckmäßigerweise eine Kondensationstemperatur von 5 bis 30 °C beim obengenannten Systemdruck eingestellt.

Der in der zweiten Kondensationsstufe anfallende Alkohol kann ohne vorhergehende Reinigung beispielsweise erneut zur Herstellung von Diurethan verwendet werden.

Das in der ersten Kondensationsstufe erhaltene Diisocyanat wird zur Reinigung üblicherweise destilliert und besitzt danach eine Reinheit von über 99,5 Gew.-%.

Nach dem erfindungsgemäßen Verfahren hergestellte Diisocyanate eignen sich vorzüglich zur Herstellung von Polyurethan- oder Polyurethan-Polyharnstoff-Kunstoffen und insbesondere für lichtbeständige Polyurethanlacke und -überzüge.

Die in den Beispielen genannten Teile sind Gewichtsteile.

### Beispiel 1

Die gesamte Spaltapparatur, die aus einem Dünnschichtverdampfer, einem Spaltreaktor und einer Zweistufen-Dampfkondensationsvorrichtung bestand, wurde auf 20 bis 22 mbar evakuiert.

Innerhalb von 4,5 Stunden wurden 1 200 Teile Hexamethylen-di-n-butylurethan als Schmelze so in den auf 260 °C erhitzten Dünnfilmverdampfer eingebracht, daß 591 Teile abliefen und 609 Teile verdampften. Die Hexamethylen-di-n-butylurethandämpfe gelangten in einen Spaltreaktor mit ca. 1 l Leervolumen, der mit Messingringen von 3 mm Durchmesser gefüllt war. Die Temperatur im Spaltreaktor betrug durchschnittlich 370 °C. Die austretenden Spaltgase wurden in der nachfolgenden zweistufigen Kondensationsvorrichtung fraktioniert kondensiert. Im ersten, bei 85 °C betriebenen Kondensator erhielt man 273 Teile Hexamethylendiisocyanat (HDI) und 17 Teile Butoxicarbonylaminohexamethylenisocyanat. Im zweiten, bei 10 bis 12 °C betriebenen Kondensator erhielt man 240 Teile n-Butanol und 68 g Hexamethylendibutylurethan, das durch Rekombination von mitgeschlepptem HDI mit Butanol entstanden war.

Die Selektivität der Spaltung betrug somit zu HDI 94,9 %, zu rückführbarem Butoxicarbonylaminohexamethylenisocyanat 4,1 % und zu Butanol 96,8 %.

### Beispiel 2

Man verfuhr analog den Angaben von Beispiel 1, stellte jedoch die folgenden Reaktionsbedingungen ein :

4

Druck :                                                                30 mbar

Verdampfertemperatur :                    265 °C

Spalttemperatur :                        380 °C

1. Kondensatortemperatur :         80 °C

2. Kondensatortemperatur :         12 °C

Reaktorfüllung :                        $V_2A$-Ringe, 5 mm

Von 1 748 Teilen des in den Dünnschichtverdampfer als Schmelze eingebrachten Hexamethylendibutylurethans wurden 1 150 Teile verdampft. Man erhielt 548 Teile Hexamethylendiisocyanat, 15,3 Teile Butoxicarbonylaminohexamethylenisocyanat, 499 Teile n-Butanol und 14,6 Teile durch Rekombination entstandenes Hexamethylendibutylurethan. Die selektivität der Spaltung zu HDI betrug 90.8 %, zu rückführbarem Butoxicarbonylaminohexamethylenisocyanat 1,8 % und zu Butanol 94,7 %.

## Beispiel 3

Die gesamte Spaltapparatur, die aus einem Dünnschichtverdampfer, einem Spaltreaktor und einer Zweistufen-Dampfkondensationsvorrichtung besteht, wurde auf 20 bis 22 mbar evakuiert.

Innerhalb von 21 Stunden wurden 5 600 Teile Hexamethylen-di-n-butylurethan als Schmelze so in den auf 260 °C erhitzten Dünnfilmverdampfer eingebracht, daß 2 738 Teile abliefen und 2 862 Teile verdampften. Die Hexamethylendi-n-butylurethan-Dämpfe gelangten in einen Spaltreaktor mit ca. 1 l Leervolumen, der mit Messingringen von 3 mm Durchmesser gefüllt war. Die Temperatur im Spaltreaktor betrug durchschnittlich 370 °C bei einem Druck von 20 bis 30 mbar. Über ein Feindosierventil wurden zusätzlich 0,263 Teile Chlorwasserstoff, vermischt mit 5 Vol.-Teilen Stickstoff, in den Spaltreaktor eingedüst. Die Austräge des ersten, bei 85 °C betriebenen Kondensators wurden alle 3 Stunden abgenommen und auf ihren Isocyanatgehalt untersucht. In der Tabelle 1 ist das Verhältnis in Teilen von Hexamethylendiisocyanat-1,6 (HDI-1,6) zu Butoxicarbonylaminohexamethylenisocyanat (« Monoisocyanat ») in Abhängigkeit von der Reaktionsdauer angegeben. Eine signifikante Verhältnisänderung ist nicht erkennbar.

### Tabelle 1

| Zeit [Std.] | 3 | 6 | 9 | 12 | 15 | 18 | 21 |
|---|---|---|---|---|---|---|---|
| HDI-1,6 [Teile] | 91,3 | 92,7 | 91,6 | 89,3 | 92,8 | 88,1 | 90,6 |
| Monoisocyanat [Teile] | 8,7 | 7,3 | 8,4 | 10,7 | 7,2 | 11,9 | 9,4 |

## Beispiel 4

Man verfuhr analog den Angaben des Beispiels 3, ohne jedoch Chlorwasserstoff in den Spaltreaktor einzudosieren. Von den 5 600 Teilen Hexamethylendi-n-butylurethan wurden 2 884 verdampft. In Tabelle 2 ist das Verhältnis von Hexamethylendiisocyanat-1,6 (HDI-1,6) zu Butoxicarbonylaminohexamethylenis ocyanat (« Monoisocyanat ») in Abhängigkeit von der Reaktionsdauer wiedergegeben. Wie man sieht, ließ nach ca. 12 Stunden die Spaltleistung nach und die Selektivität ging im Hinblick auf HDI-1,6 zurück.

### Tabelle 2

| Zeit [Std.] | 3 | 6 | 9 | 12 | 15 | 18 | 21 |
|---|---|---|---|---|---|---|---|
| HDI-1,6 [Teile] | 94,3 | 92,7 | 91,8 | 92,6 | 86,5 | 78,3 | 64,1 |
| Monoisocyanat [Teile] | 5,7 | 7,3 | 8,2 | 7,4 | 13,5 | 21,7 | 35,9 |

## Beispiel 5

Innerhalb von 5,2 Stunden wurden 1 525 Teile 2-Methyl-pentamethylen-dibutylurethan über eine auf 130 °C erhitzte Dosierkolbenpumpe in den auf 280 bis 285 °C erhitzten Dünnschichtverdampfer so eingebracht, daß 643 Teile abliefen und 882 Teile verdampften. Die Diurethandämpfe gelangten in den mit

V$_2$A-Ringen von 3 mm Durchmesser gefüllten Spaltreaktor. Die Temperatur im Spaltreaktor betrug durchschnittlich 405 °C, der Druck 20 bis 30 mbar. Über ein Feindosierventil wurden zusätzlich 0,087 Teile Chlorwasserstoff, vermischt mit 2 Volumenteilen Stickstoff, in den Spaltreaktor eingedüst. Die austretenden Spaltgase wurden in der nachgeschalteten zweistufigen Kondensationsvorrichtung fraktionierend kondensiert. Im ersten, bei 82 °C betriebenen Kondensator erhielt man 365 Teile 2-Methylpentamethylen-diisocyanat und 61,4 Teile eines Gemisches aus 2-(bzw. 4-)-Methyl-butoxicarbonylaminopentylisocyanat. Im zweiten, bei 10 bis 12 °C betriebenen Kondensator erhielt man 340 Teile n-Butanol und 112 Teile 2-Methylpentamethylendibutylurethan, das durch Rekombination von mitgeschlepptem Diisocyanat mit n-Butanol entstanden war.

Die Selektivität der Spaltung betrug somit zu 2-Methylpentamethylen-diisocyanat 90,6 %, zu rückführbarem 2-(bzw.4-)-Methyl-butoxicarbonylamino-pentylisocyanat 9,1 % und zu Butanol 99,6 %.

## Patentansprüche

1. Verfahren zur Herstellung von Hexamethylen-diisocyanat-1,6 und/oder isomeren aliphatischen Diisocyanaten mit 6 Kohlenstoffatomen im Alkylenrest durch thermische Spaltung von Hexamethylen-dialkylurethanen-1,6 und/oder isomeren aliphatischen Dialkylurethanen mit 6 Kohlenstoffatomen im Alkylenrest, dadurch gekennzeichnet, daß man die Dialkylurethane mit 1 bis 8 Kohlenstoffatomen im Alkylrest bei Temperaturen von 220 bis 300 °C und unter einem Druck von 0,1 bis 200 mbar in einem Verdampfer unzersetzt verdampft, bei Temperaturen über 300 °C in der Gasphase unter vermindertem Druck spaltet und die gebildeten Spaltgase fraktionierend kondensiert.

2. Verfahren nach Anspruch 1, dadurch gekennzeichnet, daß man die Spaltung bei Temperaturen von 310 bis 480 °C durchführt.

3. Verfahren nach Anspruch 1, dadurch gekennzeichnet, daß man die Spaltung in Gegenwart von Halogenwasserstoffen und/oder Halogenwasserstoff-Donatoren, die unter den Reaktionsbedingungen Halogenwasserstoff bilden, durchführt.

4. Verfahren nach Anspruch 1, dadurch gekennzeichnet, daß man die Spaltung in Gegenwart von Halogenwasserstoffen aus der Gruppe Bromwasserstoff oder Chlorwasserstoff durchführt.

5. Verfahren nach Anspruch 1, dadurch gekennzeichnet, daß man die Spaltung in Gegenwart von 0,001 bis 1,0 Mol-% Halogenwasserstoff, bezogen auf Hexamethylen-dialkylurethane-1,6 und/oder isomere aliphatische Dialkylurethane mit 6 Kohlenstoffatomen im Alkylenrest durchführt.

6. Verfahren nach Anspruch 1, dadurch gekennzeichnet, daß man die Spaltung in Gegenwart von Füllkörpern aus temperaturbeständigen, gasdurchlässigen Materialien durchführt.

7. Verfahren nach Anspruch 1, dadurch gekennzeichnet, daß man die Spaltung in Gegenwart von temperaturbeständigen, gasdurchlässigen Füllkörpern aus Kohle, Stahl, Messing, Kupfer, Zink, Aluminium, Titan, Chrom, Kobalt, Nickel und/oder Quarz durchführt.

8. Verfahren nach Anspruch 1, dadurch gekennzeichnet, daß man die Spaltprodukte in hintereinander geschalteten Kondensatoren durch fraktionierende Kondensation abtrennt.

9. Verfahren nach Anspruch 1, dadurch gekennzeichnet, daß man als Hexamethylen-dialkylurethane-1,6 und/oder isomere aliphatische Dialkylurethane mit 6 Kohlenstoffatomen im Alkylenrest Hexamethylen-1,6-, 2-Methylpentamethylen-1,5-, 2-Ethyl-tetramethylen-1,4-dimethylurethan, -diethylurethan, -dipropylurethan, -di-n-butylurethan, -di-iso-butylurethan, -di-n-pentylurethan und/oder -di-iso-pentylurethan verwendet.

10. Verfahren nach Anspruch 1, dadurch gekennzeichnet, daß die isomeren aliphatischen Diisocyanate mit 6 Kohlenstoffatomen im Alkylenrest aus 2-Methylpentamethylen-diisocyanat-1,5 und/oder 2-Ethyl-tetramethylen-diisocyanat-1,4 bestehen.

## Claims

1. A process for the preparation of hexamethylene 1,6-diisocyanate and/or isomeric aliphatic diisocyanates, where alkylene is of 6 carbon atoms, by thermal cleavage of hexamethylene-1,6-dialkylurethanes and/or isomeric aliphatic dialkylurethanes, where alkylene is of 6 carbon atoms, wherein the dialkylurethanes, where alkyl is of 1 to 8 carbon atoms, are vaporized, without decomposition, at from 220 to 300 °C under a pressure of from 0.1 to 200 mbar in a vaporizer and are thermally cleaved in the gas phase at above 300 °C under reduced pressure, and the gases produced in the cleavage reaction are fractionally condensed.

2. A process as claimed in claim 1, wherein the cleavage is carried out at from 310 to 480 °C.

3. A process as claimed in claim 1, wherein the cleavage is carried out in the presence of a hydrogen halide and/or a donor which forms hydrogen halide under the reaction conditions.

4. A process as claimed in claim 1, wherein the cleavage is carried out in the presence of a hydrogen halide selected from the group consisting of hydrogen bromide and hydrogen chloride.

5. A process as claimed in claim 1, wherein the cleavage is carried out in the presence of from 0.001 to 1.0 mole %, based on hexamethylene-1,6-dialkylurethanes and/or isomeric aliphatic dialkylurethanes, where alkylene is of 6 carbon atoms, of a hydrogen halide.

6. A process as claimed in claim 1, wherein the cleavage is carried out in the presence of a packing consisting of materials which are thermally stable and permeable to gas.

7. A process as claimed in claim 1, wherein the cleavage is carried out in the presence of a thermally stable packing which is permeable to gas and consists of carbon, steel, brass, copper, zinc, aluminum, titanium, chromium, cobalt, nickel and/or quartz.

8. A process as claimed in claim 1, wherein the cleavage products are separated by fractional condensation in condensers arranged in series.

9. A process as claimed in claim 1, wherein hexamethylene-1,6-, 2-methylpentamethylene-1,5-, 2-ethyltetramethylene-1,4-dimethylurethane, -diethylurethane, -dipropylurethane, -di-n-butylurethane, -diisobutylurethane -di-n-pentylurethane and/or -diisopentylurethane are used as the hexamethylene-1,6-dialkylurethanes and/or isomeric aliphatic dialkylurethanes, where alkylene is of 6 carbon atoms.

10. A process as claimed in claim 1, wherein 2-methylpentamethylene 1,5-diisocyanate and/or 2-ethyltetramethylene 1,4-diisocyanate are obtained as the isomeric aliphatic diisocyanates, where alkylene is of 6 carbon atoms.

## Revendications

1. Procédé de préparation d'hexaméthylène-diisocyanate-1,6 et/ou de diisocyanates aliphatiques isomères ayant 6 atomes de carbone dans le reste alkylène, par scission thermique d'hexaméthylène-dialkyluréthannes-1,6 et/ou de dialkyluréthannes aliphatiques, isomères, ayant 6 atomes de carbone dans le reste alkylène, caractérisé par le fait que, dans un évaporateur, on vaporise, non décomposés, les dialkyluréthannes ayant 1 à 8 atomes de carbone dans le reste alkyle, à des températures de 220 à 300 °C et sous une pression de 0,1 à 200 mbar, on les scinde sous pression réduite, dans la phase gazeuse, à des températures supérieures à 300 °C, et on condense, en les fractionnant, les gaz de scission formés.

2. Procédé selon la revendication 1, caractérisé par le fait que l'on effectue la scission à des températures de 310 à 480 °C.

3. Procédé selon la revendication 1, caractérisé par le fait que l'on effectue la scission en présence d'hydracides et/ou de donneurs d'hydracide, qui forment, dans les conditions de réaction, de l'hydracide.

4. Procédé selon la revendication 1, caractérisé par le fait qu'on effectue la scission en présence d'hydracides du groupe, acide bromhydrique ou acide chlorhydrique.

5. Procédé selon la revendication 1, caractérisé par le fait qu'on effectue la scission en présence de 0,001 à 1,0 mole% d'hydracide par rapport aux hexaméthylène-dialkyluréthannes-1,6 et/ou dialkyluréthannes aliphatiques, isomères ayant 6 atomes de carbone dans le reste alkylène.

6. Procédé selon la revendication 1, caractérisé par le fait qu'on effectue la scission en présence de masses de remplissage en matières perméables au gaz et résistantes à la température.

7. Procédé selon la revendication 1, caractérisé par le fait qu'on effectue la scission en présence de masses de remplissage perméables au gaz et résistantes à la température, en charbon, acier, laiton, cuivre, zinc, aluminium, titane, chrome, cobalt, nickel et/ou quartz.

8. Procédé selon la revendication 1, caractérisé par le fait qu'on sépare les produits de scission par condensation fractionnée dans les condenseurs disposés en série.

9. Procédé selon la revendication 1, caractérisé par le fait qu'on utilise comme hexaméthylène-dialkyluréthannes-1,6 et/ou dialkyluréthannes aliphatiques, isomères, ayant 6 atomes de carbone dans le reste alkylène, les hexaméthylène-1,6-, 2-méthyl-pentaméthylène-1,5-, 2-éthyl-tétraméthylène-1,4-diméthyluréthane, -diéthyluréthane, -dipropyluréthane, -di-n-butyluréthane, -di-iso-butyluréthane, -di-n-pentyluréthane et/ou -di-iso-pentyluréthane.

10. Procédé selon la revendication 1, caractérisé par le fait que les diisocyanates aliphatiques, isomères ayant 6 atomes de carbone dans le reste alkylène sont constitués par 2-méthylpentaméthylène-diisocyanate-1,5 et/ou 2-éthyl-tétraméthylène-diisocyanate-1,4.